(19) Europäisches Patentamt
European Patent Office
Office européen des brevets

(11) Veröffentlichungsnummer: **0 026 398**
**B1**

(12) # EUROPÄISCHE PATENTSCHRIFT

(45) Veröffentlichungstag der Patentschrift: **25.05.83**

(51) Int. Cl.³: **A 61 K 6/08**

(21) Anmeldenummer: **80105568.2**

(22) Anmeldetag: **17.09.80**

(54) **Dentalwerkstoffe auf Basis von organischen Kunststoffen in pastöser Form.**

(30) Priorität: **26.09.79 DE 2938875**

(43) Veröffentlichungstag der Anmeldung: **08.04.81 Patentblatt 81/14**

(45) Bekanntmachung des Hinweises auf die Patenterteilung: **25.05.83 Patentblatt 83/21**

(84) Benannte Vertragsstaaten: **BE CH DE FR GB IT LI LU NL SE**

(56) Entgegenhaltungen:
**EP-A-0 011 734**
**EP-A-0 011 735**
**DE-A-2 312 258**
**DE-A-2 403 211**
**FR-A-2 028 274**
**FR-A-2 182 186**
**FR-A-2 361 863**
**US-A-3 647 498**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Podszun, Wolfgang, Dr., Wolfskaul 4, D-5000 Köln 80 (DE)**
Erfinder: **Walkowiak, Michael, Dr., Albertus-Magnus-Strasse 10, D-5090 Leverkusen 1 (DE)**
Erfinder: **Schulz, Hans-Hermann, Dr., Am Neulandkreuz 23, D-5653 Leichlingen 1 (DE)**

**EP 0 026 398 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Dentalwerkstoffe auf Basis von organischen Kunststoffen in pastöser Form

Die Verwendung von gefüllten Kunststoffmaterialien als Werkstoffe für künstliche Zähne, Brücken, Kronen und Zahnfüllungen ist bekannt. Zur Herstellung von Zahnfüllungen werden diese Materialien üblicherweise in Form von Zubereitungen aus anorganischen Füllstoffen, gegebenenfalls organischen Polymerisaten und polymerisierbaren Bindern zur Anwendung gebracht.

Die bisher bekannten Materialien haben auf Grund ihrer Konsistenz und Klebrigkeit anwendungstechnische und klinische Nachteile.

Das Einbringen des Materials in die Kavität erfolgt durch Einstreichen, häufig wird die eingebrachte Masse, bedingt durch das Haften am Füllinstrument, teilweise nach dem Einfüllen in die Kavität von der Kavitätenwandung abgezogen. Diese Erscheinung ist in der Regel vom Zahnarzt nicht feststellbar und führt daher zu nicht wandständigen inkompletten Füllungen mit den bekannten Nachteilen.

Besonders nachteilig wirkt sich das verstärkte Kleben der bisher bekannten Füllmaterialien bei mehrflächigen Kavitäten aus. So ist, wie aus der Amalgamfülltechnik her bekannt, ein einwandfreies Ausfüllen der Kavität nur möglich, wenn ein Füllmaterial portionsweise eingebracht wird. Bei dieser Fülltechnik werden zunächst kleine Portionen wandständig in die Kavitätenwinkel gestopft und anschließend erst die Kavität ausgefüllt. Eine entsprechende Arbeitsweise ist mit den bisher bekannten Kunststoffmaterialien nicht möglich.

Während bei einflächigen Füllungen im Frontzahnbereich die Oberflächengestalt durch Anlegen von Matrizenbändern erzielt wird, bereitet die Formung von okklusalen Flächen mit Materialien, die eine klebende Konsistenz aufweisen, Schwierigkeiten. So war bei den bisher bekannten Materialien eine Formung der Kauflächenbezirke nur in groben Zügen möglich. Nach dem Aushärten war daher üblicherweise eine Formgebung durch rotierende Schleif- und Polierkörper erforderlich. Bekanntlich sind hierbei Verletzungen der benachbarten Schmelzbezirke in der Regel nicht zu vermeiden. Die Folgen hiervon sind Verfälschungen des Kauflächenreliefs und gegebenenfalls okklusale Störungen.

Man hat versucht, die wünschenswerte Oberflächengestalt durch Einstellung einer »schnitzbaren« Eigenschaft zu erzielen. Dieses »schnitzbare« Verhalten tritt jedoch erst dann ein, wenn schon ein gewisser Polymerisationsumsatz erreicht ist. Erfolgt in diesem Zustand die Bearbeitung des Füllmaterials, so kann ein Auf- bzw. Einreißen der Füllungsoberfläche und damit eine Schädigung der Füllung nicht ausgeschlossen werden. Diese Risse, erzielt durch »Schnitzen«, können Eintrittspforten für Microorganismen und für Farbstoffe mit den bekannten Auswirkungen sein. Darüber hinaus kann das Bearbeiten von schon anpolymerisierten Materialien zu Polymerisationsstörungen führen.

Zum Stand der Technik gehören auch bereits Dentalwerkstoffe auf Basis von polymerisierbaren Bindern und silanisierten anorganischen Füllstoffen, beispielsweise auch Glas (DE-A-2 312 258; FR-A-2 361 863). Ein Hauptnachteil derartiger Dentalmassen liegt jedoch in ihrer schlechten Verarbeitbarkeit. Es wurde nun überraschenderweise gefunden, daß durch die Verwendung von Glasperlen aus anorganischem Füllstoff erreicht wird, daß man die Dentalwerkstoffe in gleicher Weise wie konventionelle Amalgame verarbeiten kann, da derartige Dentalwerkstoffe insbesondere auch stopfbar und modellierbar sind. Mit bisher üblichen Füllstoffen läßt sich ein derartiges Eigenschaftsbild nicht erzielen.

Die Anmeldungen EP-A-11 734 (=79 104 333) und EP-A-11 735 (=79 104 333) haben bereits Dentalwerkstoffe mit einem Gehalt an vernetzten Perlpolymerisaten mit überraschend verbesserten Eigenschaften zum Gegenstand. Diese werden durch den Austausch des dort enthaltenen anorganischen Füllmaterials durch die untenstehend bezeichneten Glasperlen weiter verbessert, wie nachstehend durch die Erläuterung der Stopf- und Modelliereigenschaften gezeigt ist.

Gegenstand der Erfindung sind somit Dentalwerkstoffe in pastöser Form auf Basis von polymerisierbaren Bindern, vernetzten Perlpolymerisaten und anorganischen Füllstoffen, welche dadurch gekennzeichnet sind, daß sie aus

a) 18 bis 50 Gewichts-%, vorzugsweise 22 bis 35 Gewichts-% an polymerisierbaren Bindern,
b) 20 bis 65 Gewichts-%, vorzugsweise 25 bis 55 Gewichts-% an vernetzten Perlpolymerisaten mit einer mittleren Korngröße von 8 bis 80 μm,
c) 5 5 bis 45 Gewichts-%, vorzugsweise 10 bis 40 Gewichts-% an Glasperlen mit einer mittleren Korngröße von 5 bis 80 μm, gegebenenfalls
d) bis zu 30 Gewichts-% an anorganischem Füllstoff mit einer mittleren Korngröße von 1 nm bis 1 μm, sowie nach Bedarf
e) Startern, Lichtschutzmitteln, Inhibitoren und Farbstoffen

bestehen.

Wie schon erwähnt, lassen sich die erfindungsgemäßen Materialien in einer Konsistenz herstellen, die eine Verarbeitung ermöglicht, wie sie in der Amalgamfülltechnik gebräuchlich ist, das heißt, sie lassen sich stopfen und modellieren.

Aufgrund einer nicht klebenden, festen, stopfbaren Konsistenz gelingt es, ein- und mehrflächige Kavitäten in mehreren Portionen wandständig aufzufüllen. Durch die besondere Eigenschaft des

Materials kommt es beim portionsweisen Stopfen nicht zu Schichtenbildung, d. h. die einzelnen Portionen verbinden sich homogen miteinander.

Nach dem jeweiligen Einbringen einer Portion in die Kavität und dem Stopfen bzw. Adaptieren bleibt diese, ohne ihre Form zu verändern, am Ort liegen, d. h. sie läßt sich auch nicht elastisch deformieren.

Die Kavitätenfüllung läßt sich ferner, auf Grund der besonderen Konsistenz, mit sogenannten Amalgampistolen durchführen, ohne daß sich das Füllmaterial wieder von der Kavitätenwandung abzieht oder an der Pistolenöffnung haften bleibt.

Die erfindungsgemäßen Materialien zeigen eine durch Instrumente formbare Konsistenz schon unmittelbar nach dem Anmischvorgang. Diese Konsistenz ermöglicht es, daß nach dem Auffüllen der Kavität, mittels geeigneter Instrumente, z. B. aus Kunststoff oder aus Metall, wie sie in der Amalgamfülltechnik verwendet werden, die okklusale individuelle Kauflächengestalt geformt wird.

Die pastösen erfindungsgemäßen Dentalwerkstoffe auf Basis von organischen Kunststoffen gehen durch Aushärtung in feste Körper über, die ein zahnähnliches Aussehen zeigen und den Vorteil besitzen, gut feinschleifbar zu sein.

Zur Herstellung der erfindungsgemäßen Dentalwerkstoffe werden 18—50, vorzugsweise 22—35 Gew.-Teile polymerisierbare Binder, 20—65, vorzugsweise 25—55 Gew.-Teile vernetzte Perlpolymerisate, 5—45, vorzugsweise 10—40 Gew.-Teile Glasperlen, gegebenenfalls bis zu 30 Gew.-Teile feinteilige anorganische Füllstoffe und 0,01 bis 5 Gew.-Teile Starterzusätze zu einer Paste vermischt.

Zur Erleichterung der Pastenfertigung können Inhibitoren oder Lichtschutzmittel zugesetzt werden. Für bestimmte Indikationen kann es angezeigt sein, zusätzlich Farbstoffe zuzusetzen.

Als polymerisierbare Binder eignen sich die Ester der Methacrylsäure von einwertigen und mehrwertigen Alkoholen gegebenenfalls im Gemisch mit anderen Vinylmonomeren. Besonders günstig ist es, wenn der Gehalt an Methacrylsäureestern über 80% beträgt.

Als geeignetes Ester der Methacrylsäure seien beispielsweise aliphatische und cycloaliphatische Ester genannt, wie Methylmethacrylat, Ethylmethacrylat und Cyclohexylmethacrylat.

Besonders gut geeignet sind ferner Ester von mehrwertigen Alkoholen, mit einem Molekulargewicht von 190—10 000, insbesondere Ester von 2- und 3wertigen Alkoholen mit einem Molekulargewicht im Bereich von 190 bis 800, wie z. B. Ethylenglykoldimethacrylat, Triethylenglykoldimethacrylat, Neopentylglykoldimethacrylat oder Trimethylolpropantrimethacrylat, des weiteren Urethan und Ureidpolymethacrylate, die durch Umsetzung von Hydroxyalkylmethacrylaten bzw. Aminoalkylmethacrylaten mit Polyisocyanaten zugänglich sind, z. B. die Verbindungen

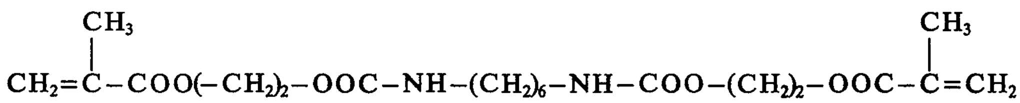

Sehr gute Pasten erhält man, wenn als Binder zumindest in Anteilen Verbindungen vom Typ des Bis-GMA der Formel

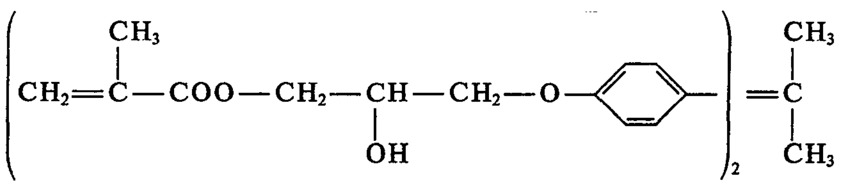

eingesetzt werden.

Zahnfüllmassen mit guter Konsistenz und einem hohen Niveau der mechanischen Festigkeit werden besonders dann erhalten, wenn man als Binder Mischungen aus verschiedenen Methacrylsäureestern verwendet, z. B. Mischungen von 20—70 Gew.-Teilen Bis GMA und 80—30 Gew.-Teilen Triethylenglykoldimethacrylat.

Die zur Pastenherstellung eingesetzten vernetzten Perlpolymerisate sollen zu mehr als 80 Gew.-% aus polymerisierten Methacrylsäureestern, vorzugsweise Methacrylsäuremethylester, bestehen. Als vernetzend wirkende Monomere eignen sich die mit Methylmethacrylat copolymerisierbaren Polyvinylverbindungen, wie beispielsweise Ethylenglykoldimethacrylat, Divinylbenzol, wobei der Vernetzeranteil 2 bis 35 Gew.-% des Monomergemisches betragen sollte. Neben dem Vernetzer können weitere Monomere in das Perlpolymerisat einpolymerisiert sein, um beispielsweise das Quellverhalten des Perlpolymerisats zu beeinflussen oder um die mechanischen Eigenschaften des ausgehärteten Dentalkunststoffs zu modifizieren. Die mittlere Korngröße der eingesetzten Perlpolymerisate soll zwischen 5 und 100 μ liegen; besonders günstig ist der Bereich von 8 bis 80 μ.

Gut geeignet für die Pastenzubereitung sind ferner Perlpolymerisate gemäß den deutschen Patentanmeldungen P 23 49 280 vom 14. 11. 78 und P 28 49 935 vom 17. 11. 78. Die Verwendung von mit anorganischen Füllstoffen gefüllten Perlpolymerisaten gemäß P 28 49 936 vom 17. 11. 78 ist besonders vorteilhaft, da auf diese Weise Dentalwerkstoffe erhalten werden können, bei denen sowohl das

Perlpolymerisat als auch die Perlzwischenräume gleichermaßen anorganischen Füllstoff enthalten.

Die verwendeten Glasperlen sollen einen mittleren Perlendurchmesser von 5 bis 80 μ, vorzugsweise von 10 bis 40 μ besitzen.

Wenn auf hohe Transparenz des ausgehärteten Dentalwerkstoffes Wert gelegt wird, müssen die Brechungsindices der Glasperlen und des Polymerisats aufeinander abgestimmt werden. Zur Herstellung eines röntgenopaken Dentalwerkstoffes können besonders vorteilhaft Gläser mit Schwermetallionen, beispielsweise Ba, La oder Zr, eingesetzt werden.

Gut geeignete Glasperlen sind zum Beispiel Refleyperlen RPG 22® der Jenaer Glaswerke Schott und Gen./Mainz.

Es ist zweckmäßig, die Glasperlen zu »silanisieren«, d. h. mit einer speziellen haftvermittelnden Silanverbindung (beispielsweise Trimethoxy-(3-methacryloyloxypropyl)-silan oder Vinyltrimethoxysilan zu behandeln, um den Verbund zwischen Glas und Kunststoffmatrix zu verbessern.

Als feinteiliger anorganischer Füllstoff für die erfindungsgemäßen Dentalmaterialien sind in erster Linie Siliziumdioxyd, Aluminiumdioxyd, Silikate und Silikatgläser geeignet, sofern ihre mittlere Teilchengrößen im Bereich von 1 mμ—1 μ liegen. Besonders günstig ist die Verwendung von flammpyrolytisch gewonnenem, amorphem Siliziumdioxyd, und zwar vorzugsweise von amorphem Siliziumdioxid mit einer Primärteilchengröße von 5—30 mμ und einer speziellen Oberfläche gemessen nach BET von 40—400 $m^2/g$.

Der feinteilige anorganische Füllstoff kann ebenso wie die Glasperlen silanisiert werden, jedoch ist dieser Nachbehandlungsschritt zur Herstellung der erfindungsgemäßen Dentalwerkstoffe nicht unbedingt erforderlich.

Zur Aushärtung der erfindungsgemäßen Dentalwerkstoffe können die üblichen Startersysteme verwendet werden, d. h. Radikale, Anionen oder Kationen liefernde Systeme, die eine radikalische, anionische oder kationische Polymerisation auslösen können. Im Falle von Radikale liefernden Systemen sind Peroxide oder aliphatische Azoverbindungen besonders geeignet, beispielsweise Benzoylperoxid, Laurylperoxid oder Azoisobuttersäuredinitril, die üblicherweise in Mengen von 0,1 bis 5 Gew.-% eingesetzt werden. Während die Härtung bei erhöhter Temperatur allein durch Peroxide oder andere Radikalstarter durchgeführt wird, ist zur Härtung bei Raumtemperatur ein Zusatz von Beschleunigern, vorzugsweise aromatischen Aminen notwendig. Geeignete Beschleuniger sind N-N-substituierte Toluidine und Xylidine, wie N,N-Dimethyl-p-Toluidin oder N,N-Bis-(2-hydroxyethyl)-xylidin. Gute Aushärtung erzielt man mit 0,5—3% Aminzusatz. Eine günstige Darbietungsform für ein Peroxid/Beschleuniger aktiviertes System ist die 2-Pasten-Form, wobei eine Paste den Radikalstarter und die andere den Beschleuniger enthält und die Aushärtung durch Mischen beider Pasten eingeleitet wird.

Auch eine Aushärtung unter Verwendung von UV-Licht oder sichtbarem Licht bei geeigneter Sensibilisierung ist eine sehr gute Methode. Geeignete Photoinitiatoren sind beispielsweise Benzophenon und seine Derivate, Benzoin und seine Derivate, wie Benzoinether, Anthrachinone und aromatische Disulfide.

## Beispiel 1

### Herstellung eines mit amorphem Siliziumdioxid gefüllten Perlpolymerisats

### Polymerisation

Reaktionsgefäß:
6-Liter-Autoklav mit Doppelankerrührer

Lösung I:
2500 ml dest. Wasser

Dispergaturlösung:
500 ml 7,5%ige wäßrige Lösung des Copolymerisats aus 1 Gew.-Teil Methacrylsäure und 1 Gew.-Teil Methylmethacrylat mit dem pH = 6 und der Viskosität: 3650 cp

Lösung II:

| | |
|---|---|
| 684 g | Methylmethacrylat |
| 36 g | Ethylenglykoldimethacrylat |
| 308 g | silanisiertes amorphes Siliziumdioxid (BET-Oberfläche 170 $m^2/g$) |
| 7,2 g | Benzoylperoxyd |

Lösung I wird vorgelegt und 5 Minuten verrührt. Bei stehendem Rührer gibt man Lösung II auf einmal zu und bespült den Autoklaven mit Stickstoff. Anschließend wird 5 bar Stickstoff aufgedrückt, die Rührergeschwindigkeit auf 400 UpM eingestellt und auf 80° C aufgeheizt. Bei einsetzender exothermer Reaktion wird so stark gekühlt, daß die Temperatur unter 90° C bleibt. Man läßt 2 Stunden bei 80° C nachrühren.

Aufarbeitung

Der Ansatz wird abgeblasen und mit dest. Wasser auf 10 l verdünnt. Nach der Zugabe von 180 g Eisessig wird 15 Minuten auf 90—100°C aufgeheizt. Das ausfallende Perlpolymerisat wird nach Erkalten abgesaugt, durch dreimaliges Aufrühren in je 5 l dest. Wasser gewaschen und bei 60°C getrocknet. Ausbeute 866 g, mittlere Perldurchmesser 45 μ.

Beispiel 2

Erfindungsgemäßer pastöser Dentalwerkstoff

A) Peroxidpaste

| | |
|---|---|
| 130 g | Perlpolymerisat aus Beispiel 1 |
| 100 g | Reflexperlen RPG 22® |
| 90 g | Bis GMA (Nupol 46—4005 der Firma Freeman Chemical) |
| 50 g | Triethylenglykoldimethacrylat |
| 2,5 g | Benzoylperoxid |

Die einzelnen Komponenten werden in einen Kneter gegeben und 60 Minuten lang intensiv geknetet, wobei während der letzten 10 Minuten ein Vakuum von ca. 20 Torr angelegt wird. Man erhält auf diese Weise eine knetbare Masse von besonders fester Konsistenz.

B) Aminpaste

Perlpolymerisat, Glasperlen, Bis GMA und Triethylenglykoldimethacrylat werden in den gleichen Mengen wie bei der Peroxidpaste (A) eingesetzt und verarbeitet. Anstelle des Peroxids werden jedoch 1,8 g N,N-Dimethyltoluidin eingesetzt.

C) Pastöse Masse zum Füllen von Zähnen

Gleiche Teile (z. B. je 200 mg) der Amin- und Peroxidpaste werden 30 Sekunden lang intensiv gemischt. Die erhaltene Mischung ist in hervorragender Weise als Zahnfüllmaterial geeignet. Sie härtet in wenigen Minuten unter geringem Polymerisationsschrumpf aus.

Beispiel 3

Erfindungsgemäßer pastöser Dentalwerkstoff

Entsprechend der in Beispiel 2 angegebenen Arbeitsweise wird aus

| | |
|---|---|
| 140 g | Perlpolymerisat gemäß Beispiel 1 |
| 80 g | Reflexperlen RPG 22® |
| 80 g | Bis GMA |
| 50 g | Triethylenglykoldimethacrylat |
| 3 g | Benzoinisopropylether |

eine pastöse Mischung hergestellt.

Dieses Material ist hervorragend als Zahnfüllungsmaterial geeignet. Es härtet unter Bestrahlung mit UV-Licht (Uviolite-Lampe der Firma Espe) innerhalb von 40 Sekunden in Schichtdicken von 2,5 mm aus.

**Patentansprüche**

1. Dentalwerkstoffe in pastöser Form auf Basis von polymerisierbaren Bindern, vernetzten Perlpolymerisaten und anorganischen Füllstoffen, dadurch gekennzeichnet, daß sie aus

a) 18 bis 50 Gewichts-%, vorzugsweise 22 bis 35 Gewichts-% an polymerisierbaren Bindern,
b) 20 bis 65 Gewichts-%, vorzugsweise 25 bis 55 Gewichts-% an vernetzten Perlpolymerisaten mit einer mittleren Korngröße von 8 bis 80 μm,

c) 5 bis 45 Gewichts-%, vorzugsweise 10 bis 40 Gewichts-% an Glasperlen mit einer mittleren Korngröße von 5 bis 80 µm, gegebenenfalls
d) bis zu 30 Gewichts-% an anorganischem Füllstoff mit einer mittleren Korngröße von 1 nm bis 1 µm, sowie nach Bedarf
e) Startern, Lichtschutzmitteln, Inhibitoren und Farbstoffen

bestehen.

2. Dentalwerkstoffe nach Anspruch 1, dadurch gekennzeichnet, daß die polymerisierbaren Binder zu mehr als 80 Gewichts-% aus Estern der Methacrylsäure bestehen.

3. Dentalwerkstoffe nach Anspruch 2, dadurch gekennzeichnet, daß die polymerisierbaren Binder zu mindestens 50 Gewichts-% aus Estern der Methacrylsäure mit zwei oder mehr polymerisierbaren Doppelbindungen bestehen.

4. Dentalwerkstoffe nach Anspruch 1 bis 3, dadurch gekennzeichnet, daß die vernetzten Perlpolymerisate zu mehr als 80 Gewicht-% aus Estern der Methacrylsäure aufgebaut sind.

5. Dentalwerkstoffe nach Anspruch 1 bis 4, dadurch gekennzeichnet, daß die vernetzten Perlpolymerisate bis zu 50 Gewichts-% an eingeschlossenen anorganischen Füllstoffen mit einer mittleren Korngröße von 1 nm bis 1 µm enthalten.

6. Dentalwerkstoffe nach Anspruch 1 bis 5, dadurch gekennzeichnet, daß die Glasperlen silanisiert sind.

7. Dentalwerkstoffe nach Anspruch 6, dadurch gekennzeichnet, daß die Glasperlen mit Trimethoxy-(3-methyl-acryloyloxy-propyl)-silan behandelt sind.

8. Dentalwerkstoffe nach Anspruch 6, dadurch gekennzeichnet, daß die Glasperlen mit Vinyltrimethoxysilan behandelt sind.

9. Dentalwerkstoffe nach Anspruch 1 bis 8, dadurch gekennzeichnet, daß die feinteiligen anorganischen Füllstoffe aus Kieselsäure bestehen.

10. Dentalwerkstoffe nach Anspruch 1 bis 9, dadurch gekennzeichnet, daß die feinteiligen anorganischen Füllstoffe silanisiert sind.

## Claims

1. Dental materials in paste form which are based on polymerisable binders, cross-linked bead polymers and inorganic fillers, characterised in that they consist of

a) 18 to 50% by weight, preferably 22 to 35% by weight of polymerisable binders,
b) 20 to 65% by weight, preferably 25 to 55% by weight of cross-linked bead polymers with an average particle size of 8 to 80 µm,
c) 5 to 45% by weight, preferably 10 to 40% by weight, of glass beads with an average particle size of 5 to 80 µm, if appropriate
d) up to 30% by weight of an inorganic filler with an average particle size of 1 nm to 1 µm, and if appropriate
e) starters, light stabilisers, inhibitors and dyestuffs.

2. Dental materials according to claim 1, characterized in that the polymerisable binders consist of esters of methacrylic acid to the extent of more than 80% by weight.

3. Dental materials according to claim 2, characterized in that the polymerisable binders consist of esters of methacrylic acid with two or more polymerisable double bonds to the extent of at least 50% by weight.

4. Dental materials according to claim 1 to 3, characterized in that the cross-linked bead polymers are built up from esters of methacrylic acid to the extent of more than 80% by weight.

5. Dental materials according to claim 1 to 4, characterized in that the cross-linked bead polymers contain up to 50% by weight of incorporated inorganic fillers with an average particle size of 1 nm to 1 µm.

6. Dental materials according to claim 1 to 5, characterized in that the glass beads are silanised.

7. Dental materials according to claim 6, characterized in that the glass beads are treated with trimethoxy-(3-methylacryloyloxypropyl)-silane.

8. Dental materials according to claim 6, characterized in that the glass beads are treated with vinyltrimethoxysilane.

9. Dental materials according to claim 1 to 8, characterized in that the fine-particled inorganic fillers consist of silicic acid.

10. Dental materials according to claim 1 to 9, characterized in that the fine-particled inorganic fillers are silanised.

**Revendications**

1. Produits dentaires, sous forme pâteuse, à base de liants polymérisables, de polymérisats en perles et réticulés et de charges inorganiques, caractérisés en ce qu'ils se composent

a) de 18 à 50% en poids, de préférence de 22 à 35% en poids de liants polymérisables,
b) de 20 à 65% en poids, de préférence de 25 à 55% en poids de polymérisats en perles réticulés avec une granulométrie moyenne de 8 à 80 µm,
c) de 5 à 45% en poids, de préférence de 10 à 40% en poids, de perles de verre présentant une granulométrie moyenne de 5 à 80 µm, éventuellement,
d) de jusqu'à 30% en poids d'une charge inorganique présentant une granulométrie moyenne de 1 nm à 1 µm, et éventuellement,
e) d'agents de déclenchement, d'agents de protection contre la lumière, d'inhibiteurs et de colorants.

2. Produits dentaires selon la revendication 1, caractérisés en ce que les liants polymérisables se composent pour plus de 80% en poids d'esters de l'acide méthacrylique.

3. Produits dentaires selon la revendication 2, caractérisés en ce que les liants polymérisables se composent pour au moins 50% en poids d'esters de l'acide méthacrylique avec deux doublesliaisons polymérisables ou plus.

4. Produits dentaires selon l'une quelconque des revendications 1 à 3, caractérisés en ce que les polymérisats en perles réticulés sont constitués pour plus de 80% en poids d'esters de l'acide méthacrylique.

5. Produits dentaires selon l'une quelconque des revendications 1 à 4, caractérisés en ce que les polymérisats en perles réticulés contiennent jusqu'à 50% en poids de charges inorganiques incluses avec une granulométrie moyenne de 1 nm à 1 µm.

6. Produits dentaires selon l'une quelconque des revendications 1 à 5, caractérisés en ce que les perles de verre sont silanisées.

7. Produits dentaires selon la revendication 6, caractérisés en ce que les perles de verre sont traitées au triméthoxy-(3-méthyl-acryloyloxy-propyl)-silane.

8. Produits dentaires selon la revendication 6, caractérisés en ce que les perles de verre sont traitées avec du vinyltriméthoxysilane.

9. Produits dentaires selon l'une quelconque des revendications 1 à 8, caractérisés en ce que les charges inorganiques finement divisées se composent d'acide silicique.

10. Produits dentaires selon l'une quelconque des revendications 1 à 9, caractérisés en ce que les charges inorganiques finement divisées sont silanisées.